(19) Europäisches Patentamt
European Patent Office
Office européen des brevets

(11) Publication number : **0 670 321 A1**

# (12) EUROPEAN PATENT APPLICATION

(21) Application number : **94400459.7**

(22) Date of filing : **03.03.94**

(51) Int. Cl.[6] : **C07D 498/14,** A61K 31/47, // (C07D498/14, 263:00, 221:00, 209:00)

(43) Date of publication of application : **06.09.95 Bulletin 95/36**

(84) Designated Contracting States : **DE FR GB IT**

(71) Applicant : **MITSUBISHI GAS CHEMICAL COMPANY, INC.**
**No. 5-2, Marunouchi 2-chome**
**Chiyoda-ku, Tokyo (JP)**

(72) Inventor : **Urakami, Teizi, c/o Mitsubishi Gas Chem. Co., Inc.**
**2-5-2, Marunouchi**
**Chiyoda-ku, Tokyo 100 (JP)**
Inventor : **Oda, Mitsunori, c/o Niigata Research Lab.**
**Mitsubishi Gas Chemical Co., Inc.,**
**182, Shinwari**
**Tayuhama, Niigata-shi, Niigata 950-31 (JP)**
Inventor : **Narita, Ryuichirou, c/o Niigata Research Lab.**
**Mitsubishi Gas Chemical Co., Inc.,**
**182, Shinwari**
**Tayuhama, Niigata-shi, Niigata 950-31 (JP)**
Inventor : **Tanaka, Akinori, c/o Niigata Research Lab.**
**Mitsubishi Gas Chemical Co., Inc.,**
**182, Shinwari**
**Tayuhama, Niigata-shi, Niigata 950-31 (JP)**
Inventor : **Iesaka, Hiroyuki, c/o Niigata Research Lab.**
**Mitsubishi Gas Chemical Co., Inc.,**
**182, Shinwari**
**Tayuhama, Niigata-shi, Niigata 950-31 (JP)**

(74) Representative : **Eidelsberg, Victor Albert et al**
**Cabinet Flechner**
**22, Avenue de Friedland**
**F-75008 Paris (FR)**

(54) **Oxazopyrroloquinoline deivatives and use thereof.**

(57) A diabetic associated diseases-curing agent containing a compound of

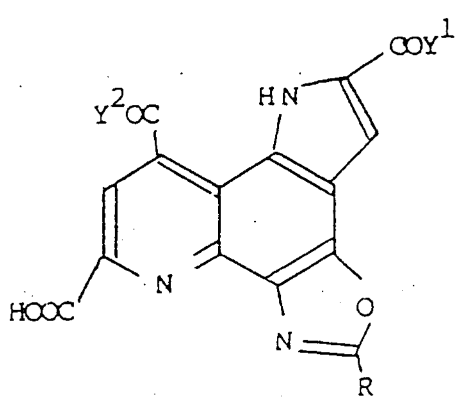

wherein R is hydrogen or $C_{1-4}$ alkyl which may be substituted, and $Y^1$ and $Y^2$ each is $OR^1$, $NHR^2$ or OH wherein $R^1$ and $R^2$ are alkyl, allyl or benzyl.

EP 0 670 321 A1

## BACKGROUND OF THE INVENTION

### Field of the Invention

The present invention relates to a diabetic associated diseases-curing agent containing as an active ingredient a novel oxazopyrroloquinoline compound. More particularly, it relates to a diabetic associated diseases-curing agent containing as an active ingredient a monoester, diester, monoamide, diamide or monoester-monoamide of 2,8,10-tricarboxy-1H-oxazo[5,4-h]pyrrolo [2,3-f]quinoline.

### Prior Art and Problem to Be Solved by the Invention

Diabetic associated diseases include diabetic cataract, diabetic neuropathy, diabetic nephropathy, diabetic retinopathy and diabetic keratitis. An aldose reductase is considered to play an important role in these diabetic associated diseases. The aldose reductase is a reducing enzyme which reduces glucose to sorbitol in the presence of NADPH. The sorbitol is converted to fructose by a dehydrogenase. The pathway in which the glucose is converted to fructose through sorbitol is called a polyol metabolism. The glucose is an important substance as an energy source. Under normal condition, after being taken into cells, almost all of the glucose is transformed into glucose 6-phosphate by an action of hexokinase, and then metabolized by glycolysis. A few percentage of the glucose is metabolized through the polyol pathway. When a hyperglycemic state occurs in diabetes, the glucose concentration in cells of insulin-nondependent tissues- such as peripheral nerves, retinae, lenses, corneas, blood vessels, renal glomeruli, and red corpuscles - increases, and glucose metabolism through the polyol pathway is accelerated, resulting in overproduction of sorbitol. The fact that the sorbitol has high polarity and is hardly diffused out of cells is considered to be the cause of the diabetic associated diseases.

Inhibition of the aldose reductase will make it possible to prevent and cure diabetic associated diseases. A variety of aldose reductase inhibitors are studied and developed. The inventors of this invention have found that oxazopyrroloquinolines (hereinafter referred to as OPQs - amino acid adducts of pyrroloquinolinequinone (PQQ), coenzyme of oxidation-reduction enzymes - and esters of OPQs have a strong aldose reductase-inhibiting activity (U.S. Patent 5,236,930). The oxazopyrroloquinolines (OPQs) include oxazopyrroloquinoline (OPQ) and 5-position-substituted oxazopyrroloquinoline. The OPQ has the formula (1) as follows:

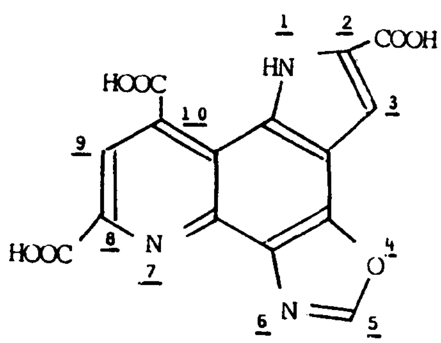

...... (1)

When these OPQs are orally applied, the OPQs administered must be easily absorbed into the body of a patient. To this effect, at least one of the three carboxyl groups in OPQs should be ester or amide forms. Although U.S. patent 5,236,930 mentions esters of OPQs, the patent is silent on structures of the esters having strong aldose reductase inhibiting activity. Therefore, there has been expectation for esters or amides of OPQs which are suitable for oral application, and have strong aldose reductase inhibiting activities.

## SUMMARY OF THE INVENTION

After the inventors of this invention conducted research, they have found that monoester, diester, monoamide, diamide or monoester-monoamide compounds of oxazopyrroloquinolines having a carboxyl group (COOH) at 8-position at the least have a small toxicity, large absorption ability in a living body and a large aldose reductase-inhibiting activity. The compounds are suitable for diabetic associated diseases-curing agents.

This invention concerns a diabetic associated diseases-curing agent, which has, as its active ingredient, monoester, diester, monoamide, diamide or monoester-monoamide compounds of oxazopyrroloquinolines represented by the formula (2).

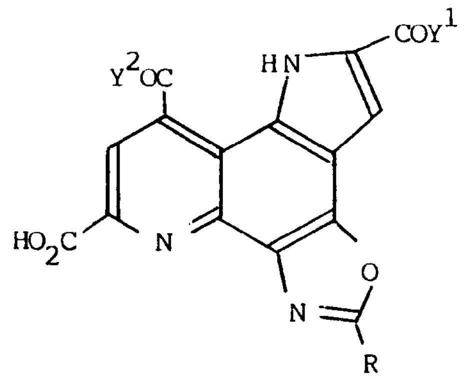

....... (2)

wherein R represents a hydrogen atom or a lower alkyl group having 1 to 4 carbon atoms which may be substituted by a substituent or substituents selected from the group consisting of hydroxyl, carboxyl, mercapto, amino, carbamoyl, phenyl, hydroxyphenyl, guanidino, imidazolyl and methylmercapto groups, and $Y^1$ and $Y^2$ each represents $OR^1$ wherein $R^1$ represents an alkyl, allyl or benzyl group, $NH\text{-}R^2$ wherein $R^2$ represents an alkyl, allyl or benzyl group, or a hydroxyl group, excluding that both $Y^1$ and $Y^2$ are hydroxyl groups.

Ester or amide compounds of OPQs are esters or amides of such OPQs as OPQ produced from PQQs and one selected from the group consisting of glycine, threonine, tryptophan, proline, tyrosine, serine and monomethylamine (JP 3-294281A); hydroxymethyl OPQ produced from PQQs and serine (JP 3-123782A); 1-methylethyl OPQ produced from PQQs and valine (JP 3-170484A); 1-methylpropyl OPQ produced from PQQs and isoleucine (JP 3-170485A); 2-methylpropyl OPQ produced from PQQs and leucine (JP 3-170486A); methyl OPQ produced from PQQs and alanine (JP 3-88081A); 2-carboxyethyl OPQ produced from PQQs and glutamic acid (JP 3-190882A); 2-carbamoylethyl OPQ produced from PQQs and glutamine (JP 3-188082A); 2-methylthioethyl OPQ produced from PQQs and methionine (JP 3-19088A); benzyl OPQ produced from PQQs and phenylalanine (JP 3-190881A); 4-hydroxyphenylmethyl OPQ produced from PQQs and tyrosine (JP 4-9387A); carboxymethyl OPQ produced from PQQs and aspartic acid; carbamoylmethyl OPQ produced from PQQs and asparagin; 4-imidazolylmethyl OPQ produced from PQQs and histidine; 4-aminobutyl OPQ produced from PQQs and lysine; 3-guanidinopropyl OPQ produced from the PQQs and arginine; and mercaptomethyl OPQ produced from PQQs and cysteine.

Examples of OPQs according to this invention are those having carboxyl groups at positions 2, 8 and 10.

Compound 1: 8,10-dicarboxy-2-methoxycarbonyl-1H-oxazo [5,4-h]pyrrolo[2,3-f]quinoline
Compound 2: 8-carboxy-2, 10-dimethoxycarbonyl-1H-oxazo [5,4-h]pyrrolo[2,3-f]quinoline
Compound 3: 10-(N-n-amyl)carbamoyl-2,8-dicarboxy-1H-oxazo[5,4-h]pyrrolo[2,3-f]quinoline
Compound 4: 10-(N-n-amyl)carbamoyl-8-carboxy-2-methoxycarbonyl-1H-oxazo[5,4-h]pyrrolo [2,3-f]quinoline

Process for producing the compounds 1-4

The OPQ esters of compounds 1 and 2 are synthesized by treating the corresponding PQQ esters with formalin/ammonium chloride. The PQQ esters are produced by selective hydrolysis of trimethyl esters of PQQ, as shown in Formula (3). Esterification of PQQ is effected by an acid catalyst reaction using alcohol, a reaction with alkyl halides and bases, or a reaction with alcohol or alkoxide and reaction auxiliaries such as 2-halopyridinium salt, dicarbonylimidazole and dicyclohexylcarbodiimide. The selective hydrolysis may be effected under basic or acidic conditions. Alternatively, the hydrolysis may be effected by reactions with reagents such as trimethylsilyl iodide, lithium iodide, halogenated aluminum/alkylthiol, boron tribromide and potassium cyanide.

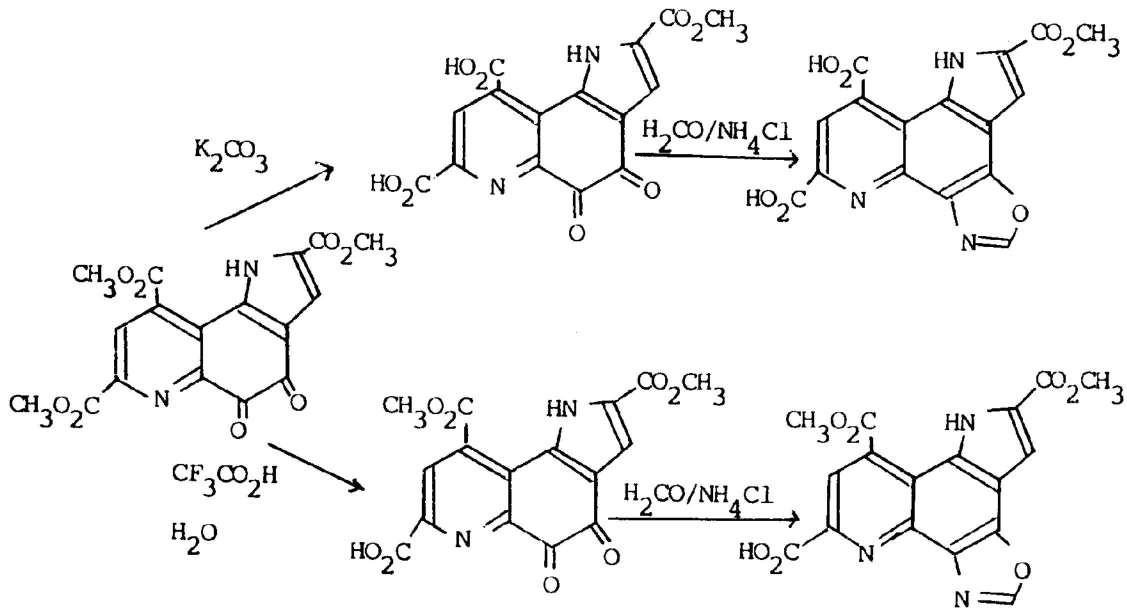


....... (3)

Compounds 3 and 4 are obtained by hydrolyzing 10-(N-n-amyl) carbamoyl-2,8-dimethoxy-carbonyl-1H-oxazo[5,4-h]pyrrolo[2,3-f]quinoline (abbreviated as OPQ-2,8-DME-10-AA). The OPQ-2,8-DME-10-AA is obtained by treating 2,7-dimethoxycarbonyl-9-carboxy-PQQ with formalin/ammonium chloride and then allowing the compound having a carboxyl group at 10-position thus obtained to react with amine in the presence of condensation reagents.

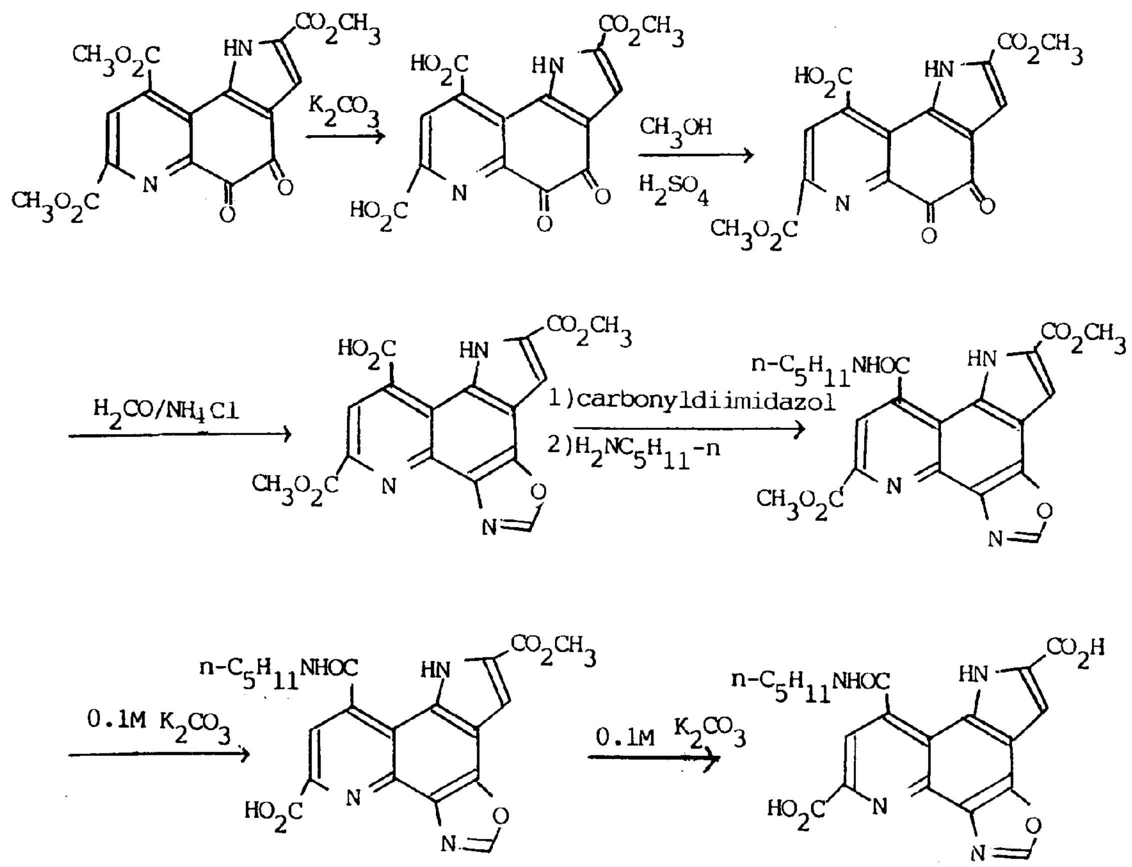


Purification of monoester, diester, monoamide, diamide or monoester-monoamide of OPQs is effected by extraction, recrystallization, silica gel chromatography, reversed-phase chromatography, gel filtration, concentration, centrifugation and/or drying. Identification of the compound thus produced is made by elemental analysis, nuclear magnetic resonance spectroscopy, infrared absorption spectroscopy, and ultraviolet-visible absorption spectroscopy.

Quntitative assay

The assay is carried out by high-speed liquid chromatography. Physical properties of these compounds are shown in Table 1.

Table 1

| Compound No. | Melting point[a)] | $^1$H-NMR spectrum ($\delta$ ppm value)[b)] |
|---|---|---|
| 1 | higher than 300°C | 3.91 (s, 3H), 7.37 (d, J=2Hz, 1H), 8.62 (s, 1H), 9.68 (s, 1H), 13.89 (br, 1H) |
| 2 | higher than 300°C | 3.99 (s, 3H), 4.20 (s, 3H), 7.43 (d, J=2Hz, 1H), 8.41 (s, 1H), 9.57 (s, 1H), 13.53 (br, 1H) |
| 3 | higher than 300°C | 0.90 (t-like, 3H), 1.1-1.6 (m, 6H), 3.47 (m, 2H), 7.29 (d, J=2.6Hz, 1H), 8.22 (s, 1H), 9.30 (s, 1H), 9.50 (br, 1H), 12.88 (br, 1H) |
| 4 | 256-260°C | 0.90 (t-like, 3H), 1.2-1.7 (m, 6H), 3.41 (m, 2H), 7.33 (d, J=2.3Hz, 1H), 8.22 (s, 1H), 9.29 (s, 1H), 13.00 (br, 1H), |

NOTE a): Decomposition observed during assay for all compounds.

NOTE b): Internal standard: Dimethyl bisulfoxide or tetramethylsilane in dimethyl biformamide

Pharmaceutical preparation

The active ingredients of this invention are formulated with surface-active agents, vehicles, colorants, preservatives and coating auxiliaries. It is also possible to use other agents in addition to these.

Dosage

Dosage of the medicine may vary depending on kind and condition of disease, varieties of ester or amine of OPQs and a manner of administration. Usually, it is within the range of 1-100 mg, preferably 5-50 mg/kg (body weight) in total a day. It may be applied once or three times a day.

Examples

Production of monoester, diester, monoamide, diamide or monoester-monoamide compound of OPQs of

this invention and curing effect of diabetic associated diseases are shown by examples. It is noted, however, that the invention is not limited to these examples.

Example 1

8,10-Dicarboxy-2-methoxycarbonyl-1H-oxazo[5,4-h] pyrrolo[2,3-f]quinoline (Compound 1)

To suspension of 2,8,10-trimethoxycarbinyl-1H-oxazo[5,4-h]pyrrolo[2,5-f]quinoline (76.6 mg. 0.200 mmol) in acetonitril (300 ml) was added aqueous 0.1M potassium carbonate solution (180 ml), while being stirred at 25°C. After being stirred for 28 minutes, 1N hydrochloric acid (30 ml) was added in order to stop the reaction. The reaction mixture was concentrated to 120 ml and precipitated solids were filtered. Recrystallization was made from dimethylformamide-isopropylether until orange crystals of the titled compound (61.2 mg) were obtained. Yield: 86.2%.

Example 2

8-Carboxy-2,10-dimethoxycarbonyl-1H-oxazo[5,4-h] pyrrolo[2,3-f]quinoline (Compound 2)

Synthesis was made from trimethyl ester of PQQ in two steps.

(1) Synthesis of 7-carboxy-4,5-dihydro-2,9-dimethoxycarbonyl-4,5-dioxo-1H-pyrrolo[2,3-f]quinoline:

A solution of trimethyl ester of PQQ (100 mg, 0.269 mmol) in a mixed solvent (5 ml) of trifluoroacetic acid and water (trifluoroacetic acid/water = 3/1) was heated at 60°C for 12 hours before cooling. Water (15 ml) was added to the solution and then extraction was made with chloroform. Extract was dried over sodium sulfate, and then the solvent was removed by distillation. The remaining orange solid was thoroughly washed with ether. It was dried under reduced pressure until reddish orange of the titled compound (64 mg) were obtained. Yield: 67%.

Physical properties are as follows.

$^1$H-NMR spectrum (internal standard: tetramethylsilane in dimethyl bisulfoxide)

δ = 3.89 (s, 3H), 4.05 (s, 3H), 7.28 (s, 1H), 8.56 (s, 1H), 12.52 (s, 1H) ppm

(2) Synthesis of 8-carboxy-2,10-dimethoxycarbonyl-1H-oxazo[5,4-h]pyrrolo[2,3-f]quinoline:

To a solution of 7-carboxy-4,5-dihydro-2,9-dimethoxycarbonyl-4,5-dioxo-1H-pyrrolo[2,3-f]quinoline (35.8 mg, 0.100 mmol) in methanol (15 ml) were added 37% formalin (5 ml) and 20% ammonium chloride solution (3 ml). The mixture was stirred at room temperature for five hours under pH 5-6 controlled with 4N sodium hydroxide. The pH of the solution was changed to 1.5 with 2N hydrochloric acid. The solution was concentrated with an evaporator to half the original volume. The precipitated solid was filtered and washed with 0.1 N hydrochloric acid, ethanol and ether. It was dried under reduced pressure to obtain orange solids of the titled compound (30.1 mg). Yield: 81.6%.

Example 3

10-(N-n-amyl)carbamoyl-8-carboxy-2-methoxycarbonyl-1H-oxazo[5,4-h]pyrrolo[2,3-f]quinoline (Compound 4)

Synthesis from trimethyl ester of PQQ was made in five steps.

(1) Synthesis of 7,9-dicarboxy-4,5-dihydro-2-methoxycarbonyl-4,5-dioxo-lH-pyrrolo[2,3-f]quinoline:

A solution of trimethyl ester of PQQ (150 mg, 0.403 mmol) in a mixed solvent of acetonitrile (75 ml) and aqueous 0.1M potassium carbonate solution (75 ml) was stirred at 25°C for four hours. The reaction mixture was controlled to pH 1 with concentrated hydrochloric acid and then was extracted with ethyl acetate. The extracted ethyl acetate was dried over sodium sulfate. The solvent was removed by distillation leaving orange solids. The orange solids were thoroughly washed with ether and dried under reduced pressure until reddish crystals of the titled compound (98 mg) were obtained. Yield: 64%.

Physical properties are as follows.

Melting point: higher than 300°C (decomposed)

$^1$H-NMR spectrum (internal standard: tetramethylsilane in dimethyl bisulfoxide):

δ = 3.87 (s, 3H), 7.27 (s, 1H), 8.61 (s, 1H), 13.49 (s, 1H) ppm

(2) Synthesis of 2,7-dimethoxycarbonyl-9-carboxy-4,5-dihydro-4,5-dioxo-lH-pyrrolo[2,3-f]quinoline:

To a solution of the monoethyl ester of PQQ (85 mg, 0.247 mmol) obtained in the step (1) in methanol (15 ml) was added dropwise concentrated sulfuric acid (0.3 ml). The mixture was heated at 60°C for four hours. After the reaction was over, pH was controlled to 5 with 0.1M potassium carbonate, and then extraction was made with ethyl acetate. The extracted ethyl acetate was dried over sodium sulfate. The solvent was removed by distillation. Red solids obtained were purified by silica gel chromatography (development solvent: ethyl acetate/acetic acid = 1/2), and recrystallized from ethyl acetate until red crystals of the titled compound (36 mg) were obtained. Yield: 41%.

Physical properties are as follows.

Melting point: 243-244°C

$^1$H-NMR spectrum (internal standard: tetramethylsilane in dimethyl bisulfoxide):

δ = 3.87 (s, 3H), 3.95 (s, 3H), 7.27 (s, 1H), 8.63 (s, 1H), 14.52 (s, 1H) ppm

(3) Synthesis of 10-carboxy-2,8-dimethoxycarbonyl-1H-oxazo[5,4-h]pyrrolo[2,3-f]quinoline:

To a solution of the dimethyl ester of PQQ (362 mg, 1.01 mmol) obtained in the step (2) in dimethyl formamide (90 ml) and methanol (200 ml) were added 37% formalin (66 ml) and aqueous 20% ammonium chloride solution (40 ml). The mixture was heated at 60°C for 19 hours, and then cooled with ice until solids were precipitated. After being filtered and washed with 0.1N hydrochloric acid, the solids were dried under reduced pressure until crude solids (280 mg) were obtained. The solids collected were recrystallized from dimethylformamide to obtain orange solids of the titled compound (221 mg). Yield: 71.8%.

(4) Synthesis of 10-(N-n-amyl)carbamoyl-2,8-dimethoxycarbonyl-1H-oxazo[5,4-h]pyrrolo[2,3-f]quinoline:

A solution of the dimethyl ester of OPQ (100 mg, 0.272 mmol) obtained in the step (3) and carbonyldiimidazole (442 mg, 2.72 mmol) in dried dimethylformaldehyde (101 ml) was heated at 65°C for 20 hours. To the reaction solution was added dropwise a solution of n-amylamine (475 mg, 5.45 mmol) diluted with dried dimethylformamide (1 ml), while the solution was cooled with ice. Stirring was made for two hours under ice-cooling. Reaction mixture was poured into 1N hydrochloric acid (100 ml) and then extraction was made with chloroform. The organic layer was washed with 1N hydrochloric acid and water and then dried over sodium sulfate. After the solvent was removed by distillation under a reduced pressure state, the residual substances were purified by silica gel chromatography (developer: chloroform/ methanol = 95/5). Recrystallization was made from chloroform/methanol until orange crystals of the titled compound (88.8 mg) were obtained. Yield: 84.7%.

Physical properties are as follows.

Melting point: 218-222°C

$^1$H-NMR spectrum (internal standard: tetramethylsilane in bichloroform):

δ = 0.98 (t-like, 3H), 1.1-2.0 (m, 6H), 3.66 (m, 2H), 3.97 (3, 3H), 4.10 (s, 3H), 7.27 (d, J=2.6Hz, 1H), 7.85 (s, 1H), 7.90 (br, 1H), 8.79 (s, 1H), 11.77 (br, 1H) ppm

(5) Synthesis of 10-(N-n-amyl)carbamoyl-8-carboxy-2-methoxycarbonyl-1H-oxazo[5,4-h]pyrrolo[2,3-f] quinoline (Compound 4):

To a solution of the compound (79.3 mg, 0.182 mmol) obtained in the step (4) in dimethylformamide (30 ml) was added aqueous 0.1M potassium carbonate solution (19 ml). After the solution was stirred at 60°C for one hour, 1N hydrochloric acid (20 ml) was added thereto. Crystals presipitated were filtered and washed thoroughly with distilled water. The crystals were dried under reduced pressure to obtain orange crystals of the titled compound (73.6 mg). Yield: 99.0%.

## Example 4

10-(N-n-amyl)carbamoyl-2,8-dicarboxy-1H-oxazo[5,4-h] pyrrolo[2,3-f]quinoline (Compound 3)

A solution of 10-(N-n-amyl)carbamoyl-8-carboxy-2-methoxycarbonyl-1H-oxazo[5,4-h]pyrrolo[2,3-f] quinoline (Compound 4) (186 mg, 0.439 mmol) obtained in example 3 in a mixed solvent of dimethylformamide (20 ml) and aqueous 0.1M potassium carbonate solution 150 ml was stirred at 30°C for 70 hours. To the reaction mixture was added concentrated hydrochloric acid (6 ml). The mixture was centrifuged to separate precipitated solids. The solids were recrystallized from dimethylformamide to produce orange crystals of the titled compound (139 mg). Yield: 77.1%.

## Example 5

A large amount of recombinant human aldose reductase was expressed in a Baculovirus-insect cell system by Nishimura et al method (Nishimura et al, J. Biol. Chem. volume 265, pages 9,788-9,792, 1990 and Biochem. Biophys. Acta. volume 1,078, pages 171-178, 1991; and Tanimoto and Nishimura, Experimental Medicine, volume 9, No. 5 (extra issue), pages 165-170, 1991). Purification was made by affinity chromatography, gel filtration and chromatofocusing until electrophoretically uniform aldose reductase was obtained.

To 0.1M phosphoric acid buffer solution (0.8 ml) was added 3mM NADPH (0.05 ml), DL-glyceral-dehyde (0.1 ml) and distilled water (0.04 ml). The mixture was left to stand at 25°C for three minutes. To the mixture was added aldose reductase solution (0.01 ml) in order to start a reaction. Measurement was made of reduction with time in absorbance of 340 nm at 25°C. The amount of enzyme that consumes 1μ mole of NADP per minute

was taken as a unit.

Enzyme activities were measured after aqueous inhibiting substance solution (0.04 ml each) having various concentrations in place of the distilled water (0.04 ml) under the same conditions as those for aldose reductase. $IC_{50}$ values that inhibit enzyme activity by 50% were calculated on the basis of enzyme activities measured in the presence or absence of the inhibitors. The inhibitors used include:

8,10-dicarboxy-2-methoxycarbonyl-1H-oxazo[5,4-h] pyrrolo[2,3-f]quinoline (Compound 1, abbreviated OPQ-8,10-DCA-2-ME);

8-carboxy-2,10-dimethoxycarbonyl-1H-oxazo[5,4-h] pyrrolo-[2,3-f]quinoline (Compound 2, abbreviated OPQ-8-CA-2,10-DME);

10-(N-n-amyl)carbamoyl-2,8-dicarboxy-1H-oxazo [5,4-h]pyrrolo[2,3-f]quinoline (Compound 3, abbreviated OPQ-2,8-DCA-10-AA);

10-(N-n-amyl)carbamoyl-8-carboxy-2-methoxy-carbonyl-1H-oxazo[5,4-h]pyrrolo[2,3-f]quinoline (Compound 4, abbreviated OPQ-8-CA-2-ME-10-AA);

2,10-dicarboxy-8-methoxycarbonyl-1H-oxazo[5,4-h] pyrrolo-[2,3-f]quinoline (abbreviated OPQ-2,10-DCA-8-ME);

10-carboxy-2,8-dimethoxycarbonyl-1H-oxazo[5,4-h] pyrrolo-[2,3-f]quinoline (abbreviated OPQ-10-CA-2,8-DME);

2,8,10-trimethoxycarbonyl-1H-oxazo[5,4-h]pyrrolo [2,3-f]-quinoline (abbreviated OPQ-2,8,10-TME);

10-carboxy-2-methoxycarbonyl-1H-oxazo[5,4-h] pyrrolo-[2,3-f]quinoline (abbreviated OPQ-10-CA-2-ME);

8,10-diethoxycarbonyl-2-methoxycarbonyl-1H-oxazo [5,4-hj]yrrolo[2,3-f]quinoline (abbreviated OPQ-2-ME-8,10-DME);

10-(N-n-amyl) carbamoyl-2,8-dimethoxycarbonyl-1H-oxazo[5,4-h]pyrrolo[2,3-f]quinoline (abbreviated OPQ-2,8-DME-10-AA); and

2,8,10-tri[(N-n-amyl)carbamoyl]-1H-oxazo[5,4-h] pyrrolo-[2,3-f]quinoline (abbreviated OPQ-2,8,10-TAA).

The results are shown in Table 2.

Table 2

| Inhibiting substance | $IC_{50}$ (μM) |
|---|---|
| Compound 1 (OPQ-8,10-DCA-2-ME) | 0.26 |
| Compound 2 (OPQ-8-CA-2,10-DME) | 0.58 |
| Compound 3 (OPQ-2,8-DCA-10-AA) | 0.26 |
| Compound 4 (OPQ-8-CA-2-ME-10-AA) | 0.19 |
| OPQ-2,10-DCA-8-ME | 2.9 |
| OPQ-10-CA-2,8-DME | 1.3 |
| OPQ-2,8,10-TME | >100 |
| OPQ-10-CA-2ME | 1.7 |
| OPQ-2ME-8,10-DEE | >100 |
| OPQ-2,8-DME-10-AA | 93 |
| OPQ-2,8,10-TAA | 77 |

Table 2 shows that, among ester and amide compounds of oxazopyrroloquinolines, those compounds which have carboxyl groups at least at 8-position -that is, monoester, diester, monoamide, diamide or monoester-monoamide compounds of oxazopyrroloquinolines represented by the Compounds 1 to 4-exhibit a strong aldose reductase inhibiting activity.

As stated above, monoester, diester, monoamide, diamide or monoester-monoamide compounds of oxazopyrroloquinolines, wherein there are a carboxyl group at 8-position and an ester or amide group at one of 2- and 10-positions at the least, have no toxicity, a large absorbability into a living body and a large aldose

reductase-inhibiting activity, and therefore are useful for prevention and curing of diabetic associated diseases.

## Claims

1. A diadetic associated diseases-curing agent which contains, as an active ingredient, a compound represented by the following formula:

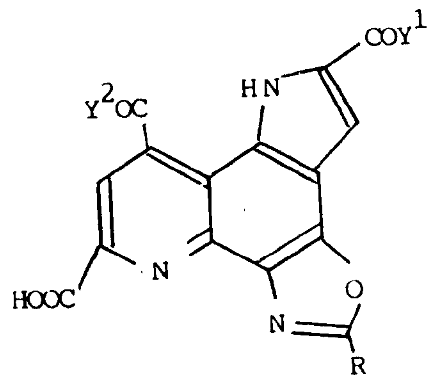

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $Y^1$ and $Y^2$ each represents $OR^1$ wherein $R^1$ represents an alkyl, allyl or benzyl group, NH-$R^2$ wherein $R^2$ represents an alkyl, allyl or benzyl group or a hydroxyl group excluding that both $Y^1$ and $Y^2$ are hydroxyl groups.

2. A diadetic associated diseases-curing agent according to claim 1 which contains, as an active ingredient, a compound represented by the following formula:

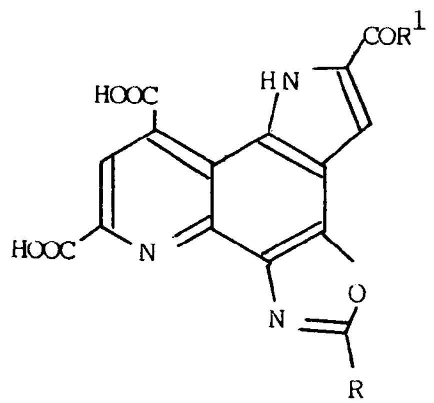

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

3. A diadetic associated diseases-curing agent according to claim 1 which contains, as an active ingredient, a compound represented by the following formula:

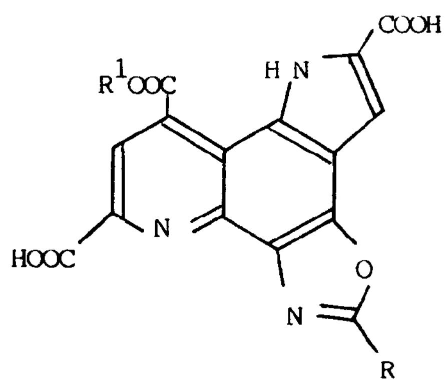

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

4. A diadetic associated diseases-curing agent according to claim 1 which contains, as an active ingredient, a compound represented by the following formula:

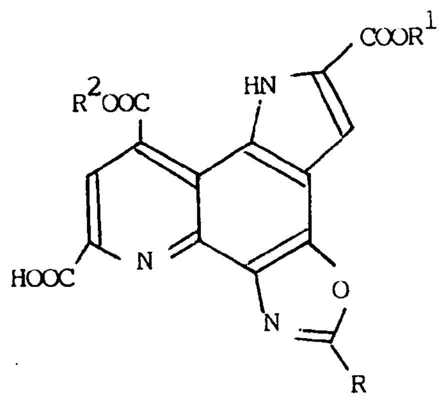

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

5. A diadetic associated diseases-curing agent according to claim 1 which contains, as an active ingredient, a compound represented by the following formula:

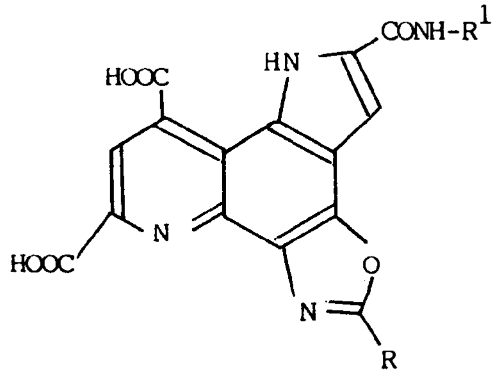

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

6. A diadetic associated diseases-curing agent according to claim 1 which contains, as an active ingredient, a compound represented by the following formula:

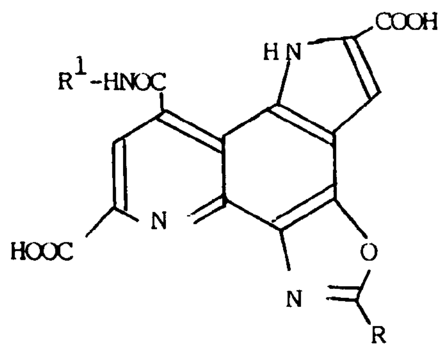

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

7. A diadetic associated diseases-curing agent according to claim 1 which contains, as an active ingredient, a compound represented by the following formula:

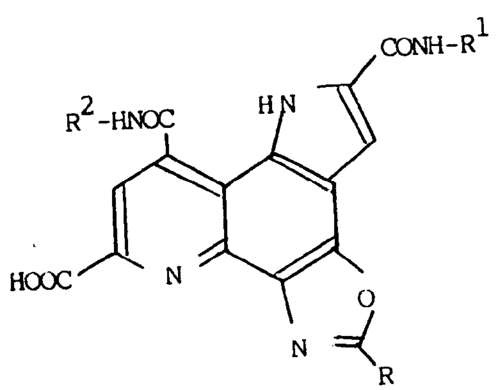

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

8. A diadetic associated diseases-curing agent according to claim 1 which contains, as an active ingredient, a compound represented by the following formula:

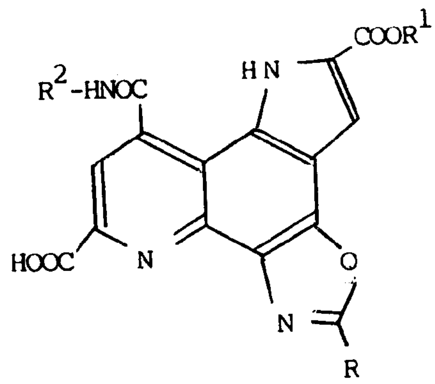

wherein R represents a hydrogen atom or a lower alkyl group having 1 ro 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

**9.** A diadetic associated diseases-curing agent according to claim 1 which contains, as an active ingredient, a compound represented by the following formula:

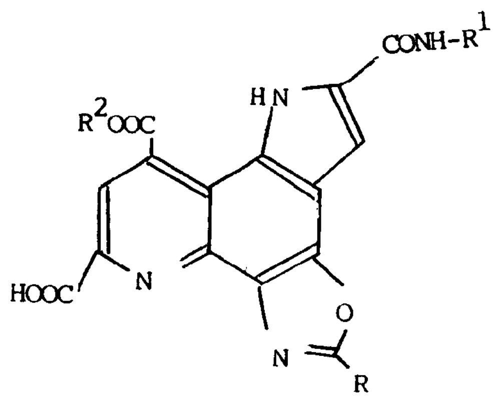

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

**10.** An aldose reductase inhibitor containing, as an active ingredient, a compound represented by the formula:

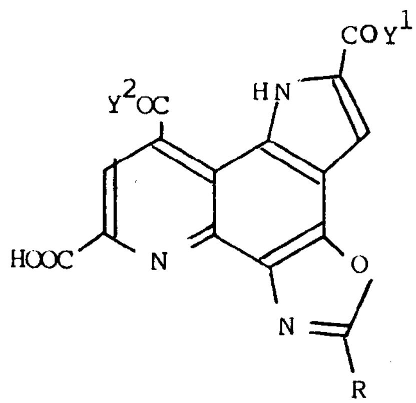

wherein R represents a hydrogen atom or a lower alkyl group having 1 to 4 carbon atoms which may be

substituted by a substituent or substituents selected from hydroxyl, carboxyl, mercapto, amino, carbamoyl, phenyl, hydroxyphenyl, guanidino, imidazolyl and methylmercapto groups, and $Y^1$ and $Y^2$ each represents $OR^1$ wherein $R^1$ represents an alkyl, allyl or benzyl group, NH-$R^2$ wherein $R^2$ represents an alkyl, allyl or benzyl group, or a hydroxyl group, excluding that both $Y^1$ and $Y^2$ are hydroxyl groups.

**11.** An aldose reductase inhibitor according to claim 10 containing, as an active ingredient, a compound represented by the formula:

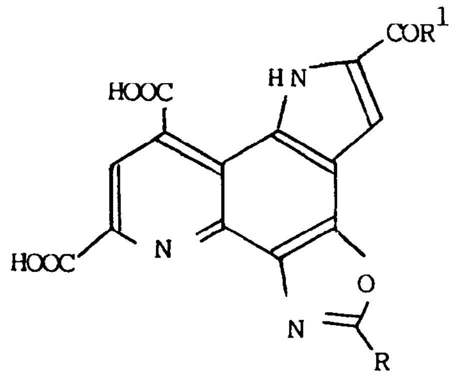

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

**12.** An aldose reductase inhibitor according to claim 10 containing, as an active ingredient, a compound represented by the formula:

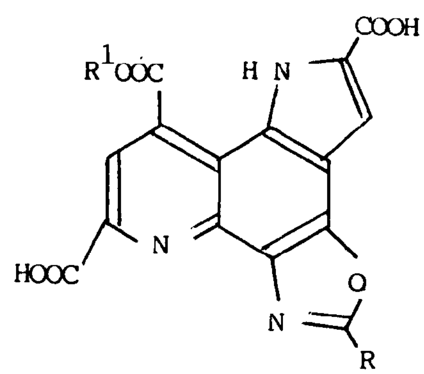

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

**13.** An aldose reductase inhibitor according to claim 10 containing, as an active ingredient, a compound represented by the formula:

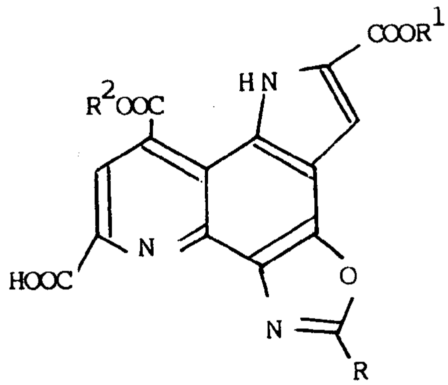

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

**14.** An aldose reductase inhibitor according to claim 10 containing, as an active ingredient, a compound represented by the formula:

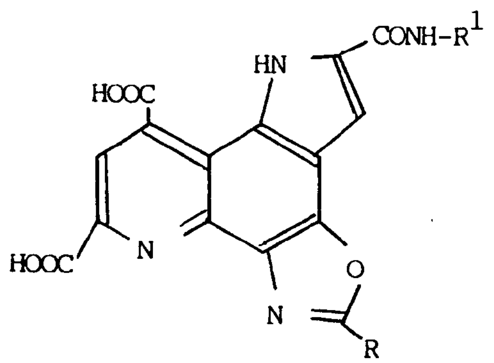

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

**15.** An aldose reductase inhibitor according to claim 10 containing, as an active ingredient, a compound represented by the formula:

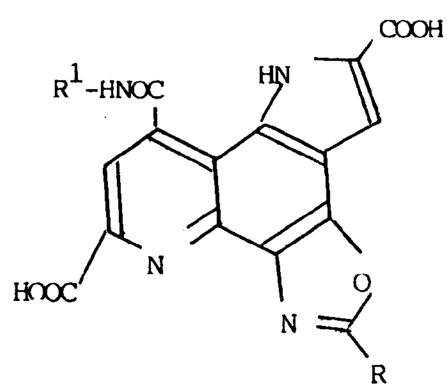

wherein R represents a hydrogen atom or a lower alkyl group having 1 to 4 carbon atoms which may be

substituted by a substituent or substituents selected from hydroxyl, carboxyl, mercapto, amino, carbamoyl, phenyl, hydroxyphenyl, guanidino, imidazolyl and methylmercapto groups, and $R^1$ represents an alkyl, allyl or benzyl group.

16. An aldose reductase inhibitor according to claim 10 containing, as an active ingredient, a compound represented by the formula:

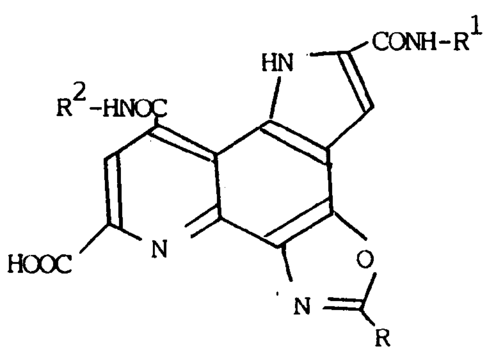

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

17. An aldose reductase inhibitor according to claim 10 containing, as an active ingredient, a compound represented by the formula:

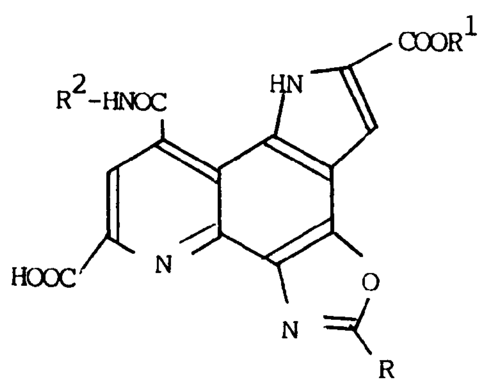

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

18. An aldose reductase inhibitor according to claim 10 containing, as an active ingredient, a compound represented by the formula:

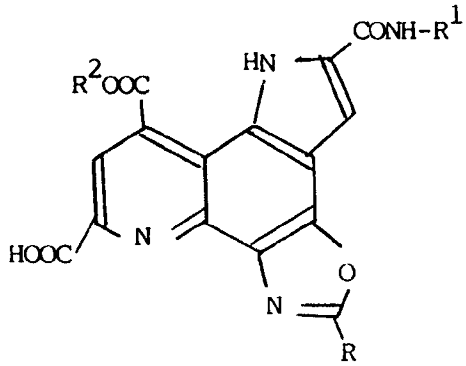

wherein R represents a hydrogen atom or a lower alkyl group having 1 to 4 carbon atoms which may be substituted by a substituent or substituents selected from hydroxyl, carboxyl, mercapto, amino, carbamoyl, phenyl, hydroxyphenyl, guanidino, imidazolyl and methylmercapto groups, and $R^1$ and $R^2$ each represents an alkyl, allyl or benzyl group.

European Patent Office

# EUROPEAN SEARCH REPORT

Application Number
EP 94 40 0459

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 555 149 (MITSUBISHI GAS CHEMICAL CO., INC. / SAGAMI CHEMICAL RESEARCH CENTER)<br>page 22, lines 17-22; Examples 12, 15 * | 1,4,10,<br>13 | C07D498/14<br>A61K31/47<br>//(C07D498/14,<br>263:00,221:00,<br>209:00) |
| A<br><br><br>D | EP-A-0 429 333 (MITSUBISHI GAS CHEMICAL CO., INC.)<br>* claims 16-19 *<br>& US-A-5 236 930 | 1,10 | |
| A | PATENT ABSTRACTS OF JAPAN<br>vol. 17, no. 567 (C-1120) 14 October 1993<br>& JP-A-05 163 279 (MITSUBISHI GAS CHEM CO INC) 29 June 1993 | 1,10 | |
| A,D | PATENT ABSTRACTS OF JAPAN<br>vol. 15, no. 413 (C-0877) 22 October 1991<br>& JP-A-03 170 484 (MITSUBISHI GAS CHEM CO INC) 24 July 1991 | 1,10 | |
| A,D | PATENT ABSTRACTS OF JAPAN<br>vol. 15, no. 413 (C-0877) 22 October 1991<br>& JP-A-03 170 485 (MITSUBISHI GAS CHEM CO INC) 24 July 1991 | 1,10 | **TECHNICAL FIELDS SEARCHED (Int.Cl.6)**<br>C07D<br>A61K |
| A,D | PATENT ABSTRACTS OF JAPAN<br>vol. 15, no. 413 (C-0877) 22 October 1991<br>& JP-A-03 170 486 (MITSUBISHI GAS CHEM CO INC) 24 July 1991 | 1,10 | |
| A,D | PATENT ABSTRACTS OF JAPAN<br>vol. 15, no. 440 (C-0883) 11 November 1991<br>& JP-A-03 188 082 (MITSUBISHI GAS CHEM CO INC) 16 August 1991<br>---<br>-/-- | 1,10 | |

The present search report has been drawn up for all claims

| Place of search | Date of completion of the search | Examiner |
|---|---|---|
| BERLIN | 27 July 1994 | Hass, C |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 40 0459

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A,D | PATENT ABSTRACTS OF JAPAN vol. 15, no. 445 (C-0884) 13 November 1991 & JP-A-03 190 881 (MITSUBISHI GAS CHEM CO INC) 20 August 1991 --- | 1,10 | |
| A,D | PATENT ABSTRACTS OF JAPAN vol. 15, no. 445 (C-0884) 13 November 1991 & JP-A-03 190 882 (MITSUBISHI GAS CHEM CO INC) 20 August 1991 --- | 1,10 | |
| A,D | PATENT ABSTRACTS OF JAPAN vol. 16, no. 156 (C-0930) 16 April 1992 & JP-A-04 009 387 (MITSUBISHI GAS CHEM CO INC) 14 January 1992 ----- | 1,10 | |
| | | | TECHNICAL FIELDS SEARCHED (Int.Cl.6) |

The present search report has been drawn up for all claims

| Place of search | Date of completion of the search | Examiner |
|---|---|---|
| BERLIN | 27 July 1994 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)